# EUROPEAN PATENT APPLICATION

(11) **EP 1 657 550 A1**
(43) Date of publication of application: **17.05.2006**
(21) Application number: 04447250.4
(22) Date of filing: 18.11.2004
(51) Int. Cl.: G01N 33/558, C12Q 1/68

(54) **Double-sided device for multiplex dipstick immunodiagnostic**

(30) Priority: 10.11.2004 EP 04447246
(71) Applicant: Coris Bioconcept SPRL, 5032 Gembloux (BE)
(72) Inventor: Leclipteux, Thierry, 5100 Wepion (BE); Degallaix, Stéphane, 5330 Maillen (BE); Denorme, Laurence, 5032 Isnes (BE); Mertens, Pascal, 5300 Seilles (BE); Olungu, Christine, 1170 Brussels (BE)
(74) Representative: Van Malderen, Joelle

(57) **Abstract**

The present invention relates to methods and devices for detecting one or more analytes or analyte products in a biological sample. This invention in particular relates to improved rapid tests such as "dipsticks" and "lateral flow" devices. The invention in particular relates to chromatographic device made of two active sides, in order to allow multiplex detections, quantitative or semi-quantitative detections, use of multiple sizes and kinds of particles or microspheres, use of different nitrocellulose porosities, use of different kinds of membranes. The devices described in this invention allow to detect or identify various biologicals or chemicals with one manipulation.

## Description

### Field of the invention.

This invention relates to methods and devices for detecting analytes or analyte analogues in a biological sample. This invention relates to rapid tests and typical rapid tests including "dipstick" and "lateral flow" devices.

### Background of the invention.

Several approaches have been developed for detection of analytes or analyte analogues in a biological sample for routine diagnostics in diagnostic laboratories via for instance immunochromatography.

EP 0 088 636, EP 0 186 799, EP 0 284 232 and WO 88/08534 disclose sheet-like chromatographic devices comprising at least a first and a second zone or region. Prior art devices disclosed in these documents comprise:
a first region or zone containing porous active material to allow liquid to move to the sensitized region coated with specific reagents. This first zone or region comprises a detection reagent dried on it or impregnated into it. It may further contain an application (sub)zone and/or an absorption (sub)zone. This first zone is generally referred to as the application zone;
a second region or zone, also referred to as the detection zone, made of porous active material on which specific reagents are adsorbed. Some of these reagents sprayed onto a subzone (e.g. a line) of the second region of the device are directly or indirectly specific for the analyte to be detected and should react with the sample analyte-labeling reagent complex while other non-specific reagents eventually sprayed onto a further subzone (e.g. as a further line) of the second region are dedicated to react with the excess of the detection reagent. This second zone or region, preferably made out of nitrocellulose, may also contain a control subzone, preferably behind the detection zone; and
a third region or region made of porous material dedicated to absorb excess of liquid coming through the first and second regions. This region is generally referred to as the absorbent or absorption region.

The (immuno)chromatographic devices of the prior art may have a plastic or other backing support and/or may be comprised in a water-impervious housing.

Several other detection techniques known in the art relate to detection of molecules produced after a preliminary process like molecular or genetic amplification of an analyte or analyte analogue.

### Aims of the invention

The present invention aims to provide highly flexible sheet-like chromatographic devices suitable for the detection of multiple analytes or analyte analogues in a solution or biological sample, these detections being carried out on the same device.

### Summary of the invention

One aspect of the present invention concerns a double-sided sheet-like chromatographic device (1), in particular a dipstick or lateral flow device, with on both sides (side #1 and side #2) of a rigid solid support (2) an application zone or region (3, 3') optionally with conjugate pad or membrane, a detection zone or region (4, 4') possibly with a control subzone or subregion, and optionally an absorbent zone or region (5, 5'). The detection zone or region of both sides (4, 4') comprises at least one, preferably several capture reagents (6, 6'). These capture reagents (6, 6') specifically recognize analytes or analyte analogues to be detected in the test sample or recognize only reference conjugates (12 - see infra). Accordingly, the application zones (3, 3') comprises at least one, preferably several analyte specific conjugates (7, 7') with direct or indirect label which allow detection of said analytes or analyte analogues and/or at least one, preferably several reference conjugates (12).

According to an embodiment of the invention, both sides (side #1 and side #2) of the device (1) comprise analyte specific capture reagents and analyte specific conjugates (7, 7'). Both sides or test sides in this case. According to another embodiment of the invention, one side is a test side and the other side a control side with capture reagents specific for reference conjugates that allow analyte or analyte analogue quantification. According to yet another embodiment, one or both sides of the device comprise both analyte specific capture reagents and capture reagents specific for reference conjugates. In a preferred embodiment, 1 side, side #1 for instance, is a test side that comprises reference conjugates to allow analyte or analyte analogue quantification. In case of a positive reaction, i.e. in case one or more complexes are formed between said analyte specific conjugates (7, 7') and said analytes, a specific signal is generated at the detection zone (4, 4') where a capture reagent (6, 6') is deposited that specifically recognizes said complex. In a preferred embodiment, reaction between capture reagents (6) and reference conjugates (12) gives rise to signals generated at the detection zone (4).

The device of the invention preferably is an immunochromatographic device.

According to an embodiment of the invention, a control line with migration control capture reagent is present on at least one side (side #1 or side #2) of the double-sided sheet-like device (1) according to the invention. Accordingly, a migration capture reagent (8) and migration (control) conjugate (9) are present on at least one side of the device (1).

In a preferred embodiment, the detection zone or region (4, 4') of at least one side further comprises one or more control capture reagents; and the application zone of said at least one side (3, 3') further comprises one or more control conjugates.

Advantageously, the control conjugate present in the double-sided sheet-like device (1) of the present invention does not interfere with the detection of the analytes or analyte analogues suspected to be present, and generates a control signal that is independent of the specific signal.

In a preferred embodiment, a signal generated by the reference control conjugates (12) on one side of the device can be used to quantify or semi-quantify analytes or analytes analogues detected on the other side of said same device (1) .

A control line, and especially a migration control line, could be present on both sides (side #1 and side #2) of the device (1) according to the invention, but is present at least on one side.

Accordingly, an embodiment of the invention concerns double-sided sheet-like device of the invention, comprising a control line with migration control capture reagent on both sides (side #1 and side #2) of the device (1).

In a preferred embodiment, the migration control capture reagent is an immunoreagent directed against a peptide or hapten coupled to or conjugated with a direct label to form a migration control conjugate. The migration control capture reagent can, however, also be neutralite avidine for instance, recognizing for instance a biotinylated migration control conjugate.

According to an embodiment of the invention, the detection region (4, 4') of both sides of the device (1) is sensitized with test antibodies and with migration control capture reagent(s), wherein the test antibody is directed against the analyte to be detected in the sample, and wherein the migration control capture reagent is directed either against an anti-analyte antibody that is coupled to a direct label to form an analyte specific conjugate (7, 7'), either against a specific conjugate non relevant to the analytes or analyte analogues to be detected.

The capture reagents (6, 6', 8) preferably are immunoreagents such as a polyclonal or monoclonal antibody or a hypervariable antibody fragment, or such as an antigen recognized by serological compounds such as IgG, IgA and IgM raised after an infection by a pathogen.

The detection label or the label of the test, migration control and/or reference control conjugates, preferably is a direct particulate label, in particular a direct label selected from the group consisting of conjugated metallic colloids, conjugated latex particles and microparticles with a particular color.

The present invention in particular relates to double-sided chromatographic devices (1) composed of polymeric substances laminated on both sides id est on opposite sides (side #1 and side #2) of a rigid polymer (2) in such a way that the said device (1) presents two active faces, preferably two differential active faces. Such devices advantageously allow the detection of different analytes or analyte analogues, which could react differently on the active membrane, on one single double-sided strip by opposition to detections performed on separate strips.

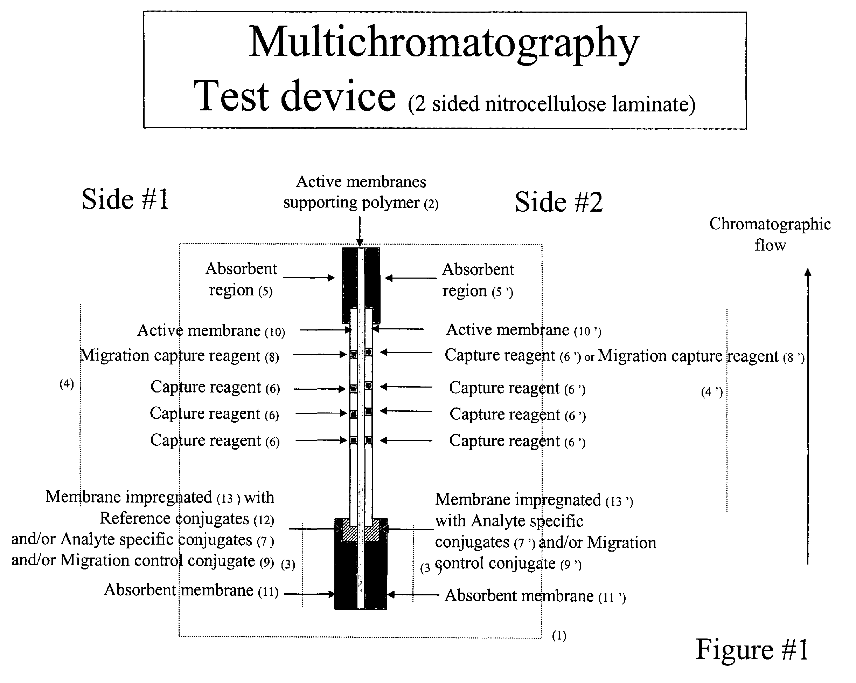

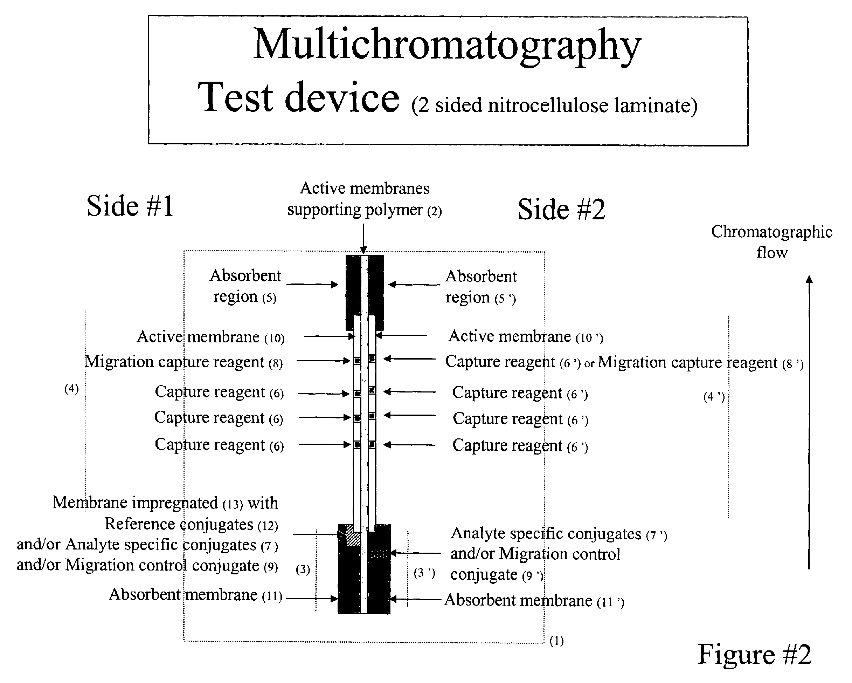

Preferably, the membranes of the application zones or regions (3, 3') are made of glass fibres with conjugate pads made of polyester (exemplified for instance in Figure 1), the membranes of the detection zone or region (4, 4') are made of nitrocellulose and the membranes of the absorbent zones or regions (5, 5') are made of cellulose. The application zones or regions (3, 3') and conjugate pads may be made of the same material. Alternatively, the conjugates may be impregnated directly onto the application zones or regions (3, 3') (exemplified for instance in Figure 2 - 3' therein).

The devices (1) of the invention are highly suitable for the detection of several analytes or analyte analogues potentially present in a test sample such as a solution or biological sample. The analytes or analyte analogues may be obtained from or may be produced by (harmful) microorganisms such as *Cryptosporidium parvum, Toxoplasma gondii, Giardia lamblia,* E. coli, RSV (Respiratory Syncytial Virus), Respiratory Adenovirus, Influenza-A and -B viruses, Rotavirus, Adenovirus type 40/41 or other Adenovirus groups.

In a preferred embodiment of the invention, more than one analyte or more than one analyte product is detected with one single double-sided device (1) according to the invention. The double-sided devices (1) of the present invention are highly suitable for multiplex detection. For instance, it is possible to detect the presence of Influenza A, Influenza B, Adenovirus and RSV on the same device (1). Rotavirus, enteric Adenoviruses, *C. parvum* and *G. lamblia* are other examples. A single (double-sided) device is more easy to handle than separate strips, especially for detection of multiple (6 to 8 or more) analytes or analyte analogues.

A particular embodiment of the invention concerns a sheet-like immunochromatographic device (1) comprising on both sides (side #1 and side #2) of a rigid solid support (2) an application zone or region (3, 3') optionally with conjugate membrane (exemplified for instance in Figure 1), a detection zone or region (4, 4') possibly with a control subzone or subregion), and optionally an absorbent zone or region (5, 5'). The detection region (4, 4') of both sides (side #1 and side #2) of the device (1) is sensitized with a test antibody. At least one of the sides (side #1 and/or side #2) is sensitized with a control antibody. According to an embodiment of the invention, both sides (sides #1 and side #2) are sensitized with at least one migration control antibody.

According to a particular example, the test antibodies of a device with control antibodies on at least one, possibly on both sides of said device, are directed against the analyte to be detected in the sample. The migration control antibodies are directed either against an anti-analyte antibody that is coupled to a direct label to form an analyte specific conjugate (7, 7'), either against a specific conjugate non relevant to the analytes or analyte analogues to be detected. The person skilled in the art is aware of the different possibilities that exist with respect to migration controls. Some particular examples are given in the Examples.

Preferably, the specific conjugate (7, 7') is deposited in the upper part of an application membrane of the sample application region (3, 3').

Preferably, the control antibody or antibodies, in particular the migration control antibodies, are deposited in a control region of the detection region, said control region being positioned above a test region in which a test antibody, in particular an analyte specific antibody is deposited. Advantageously, a signal generated at the level of the control region indicates the correct use and good condition of the sheet-like immunochromatographic device (1) according to the invention, the quality of the dried specific conjugates, as well as the completion of the immunological reaction.

Advantageously, the analyte(s) detected on side #1 is (are) different from the analyte(s) detected on side #2.

As indicated above, the test and control antibodies may be polyclonal or monoclonal antibodies or a may be a hypervariable antibody fragment. The label of the test or specific conjugates (7, 7') and of the control conjugates (9, 9', 12) may be a direct label, in particular a direct label that is selected from the group consisting of conjugated metallic colloids, conjugated latex particles and microparticles having a specific particular color.

Another aspect of the present invention concerns a double-sided sheet-like chromatographic device (1), in particular a dipstick or lateral flow device, with on both sides (side #1 and side #2) of a rigid solid support (2) an application zone or region (3, 3') optionally with conjugate pad or membrane, a detection zone or region (4, 4') possibly with a control subzone or subregion, and optionally an absorbent zone or region (5, 5'). Side #2 of said device is a test side, the detection zone (4') of side #2 comprising several capture reagents (6') specifically recognizing analytes or analyte analogues to be detected in the assay sample, the application zone (3') of said side #2 comprising several analyte specific conjugates (7') with direct or indirect label which allow detection of said analytes or analyte analogues. Side #1 of said device (1) is a control side, the detection zone (4) of said side #1 comprising at least one migration control capture reagent (8) specific for the migration control conjugate (9) with direct or indirect label comprised in the application zone (3) of said side #1. A migration control signal generated at the detection zone of side #1 indicates that the sample has migrated on both sides (side #1 and side #2) of the (immuno) chromatographic device (1) according to the invention. Advantageously, the control side (side #1) may further comprise reference capture reagents and reference control conjugates which allow quantification or at least semi-quantification. The test side (side #2) may also contain a migration control.

Advantageously, the control conjugates (migration and possibly reference controls) present in the device (1) of the present invention do not interfere with the detection of the analytes or analyte analogues suspected to be present. The reference control (see infra) advantageously generates a signal with constant intensity that is independent of the specific signal. Said control(s) may allow to quantify or semi-quantify the analyte(s) or analyte analogue(s) detected.

Preferably, the chromatographic device (1) with a test side on one side (side #1) and a control side on the other side (side #2) of a same device (1) is an immunochromatographic device (1).

The device (1) with a test side on one side (side #1) and a control side on the other side (side #2) of a same device (1) is not an oligochromatographic device as disclosed in International Patent application PCT/BE04/000061.

According to an embodiment of the invention, the migration control on the test side of such a device (1) is not comprised of an oligonucleotide deposited at the upper part of a detection region (4) but preferably is an antibody recognizing for instance a hapten coupled to or conjugated with a direct or indirect label to form a control conjugate (9).

Another embodiment of the invention relates to a double-sided sheet-like chromatographic device (1), in particular a dipstick or lateral flow device, with on both sides of a rigid solid support (2)
- an application zone or region (3, 3') optionally with conjugate pad or membrane,
- a detection zone or region (4, 4') possibly with a control subzone or subregion,
- and optionally an absorbent zone or region (5, 5') ;
wherein side #2 of said device is a test side, the detection zone (4') of which comprises one capture reagent (6') specifically recognizing one analyte or analyte analogue to be detected in the assay sample; and the application zone (3') of which comprises one specific conjugate (7') with direct or indirect label which allow detection of said analyte or analyte analogue; and wherein side #1 of said device (1) is a control side, the detection zone (4) of which comprises at least
- one migration control capture reagent (8) specific for the migration control conjugate (9) with direct or indirect label comprised in the application zone (3) of said side #1, said device (1) being an immunochromatographic device, and
- at least two, preferably at least three or four, reference capture reagents (6) specific for their relative conjugates (12) with direct or indirect label comprised in the application zone (3) of said side #1, said device being an immunochromatographic device (1),
wherein these reference reagents will give rise to signals of different intensity to be compared to the signal of the specific analyte or analyte analogue detected on side #2 of said device (1) for quantification purposes. This allows to quantify or semi-quantify with one manipulation and with one single (double-sided) device an analyte or analyte analogue by opposition to what can be observed by using separate strips. Alternatively, the test side may comprise more than one capture reagent (6') specifically recognizing one analyte or analyte analogue to be detected in the assay sample; and more than one specific conjugate (7') with direct or indirect label which allow detection of said analyte or analyte analogue.

As such, a double-sided chromatographic device according to the invention (any one of the devices described thus far) may detect analytes or analyte analogues at both (opposite) sides (side #1 and side #2) or may detect analytes or analyte analogues only on one side, the other side containing a migration control and possibly also one or more reference reagents that serve to quantify or at least semi-quantify the amount of analytes or analyte analogues present in a sample. A migration capture reagent is present on at least one side, and possibly on both sides of the device (1).

A double-sided sheet-like chromatographic device (1) according to the invention is easy to use and handle and is highly flexible.

Advantageously, the two sides (side #1 and side #2) of the double-sided chromatographic device (1) of the invention are not identical.

The double-sided chromatographic devices (1) of the invention allow for instance the use of different kinds of particles and/or different kinds of nitrocellulose's porosity and/or different kinds of sample and/or conjugates pads on the same device (1) to provide devices (1) which are easy to handle and which allow rapid but accurate detection and/or diagnosis of multiple analytes in a test sample (multiplex detection).

A particular embodiment of the invention relates to a double-sided chromatographic device (1) according to the invention wherein the porosity of the active membranes (10 and 10') of the detection zone (nitrocellulose laminates e.g.) of both sides (4 and 4') is different. For instance, the porosity of the active membrane of the detection zone of side #1 is 8 *µ*M and that of side #2 is 15 *µ*M. The person skilled in the art is aware of other possible combinations: any of a porosity of 8, 10, 12, 15 *µ*M etc may be chosen. A different porosity may be obtained by choosing for instance nitrocellulose's with a different porosity, or may be obtained by choosing two materials with each another porosity. This is technically impossible to achieve on a single-sided strip or device.

Another embodiment of the present invention concerns a double-sided chromatographic device (1), wherein the active membranes (10, 10') of both (opposite) sides are made of different materials, with a similar or a different porosity. For instance, the active membrane of one side (side #1) may be comprised of nitrocellulose and that of the other side (side #2) of Predator^{™} (Pall). The person skilled in the art is aware of other possible combinations. Possible active matrices or membranes (10, 10') for use in the detection region or zone (4, 4') include: cellulose, nitrocellulose, cellulose acetate, glass fibres, nylon, acrylic copolymer/nylon, polyethersulfone, polyethylene and polyester. This is technically impossible to achieve on single-sided devices.

Another embodiment of the present invention concerns a double-sided chromatographic device (1), wherein the conjugates dried on one side (side #1) of the device (1) bear a label that is different from the label of conjugates dried on the other side (side #2) of said same device (1). For instance, conjugates with gold particle are dried on one side (side #1) of the device (1) and conjugates with latex microspheres on the other side (side #2) of said same device (1) . The use of different types of labels in a single-sided device is technically extremely difficult especially for multiplex detection purposes. A particular embodiment of the invention relates to a device wherein a mix of conjugates (7, 9, 12) with different labels is dried on at least one side of the device (1), possibly on both sides of said device (1) . The conjugates (7, 9, 12) dried on one side of the device (1) can bear different labels, which could be different from the labels of the conjugates (7', 9') dried on the other side of said same device (1). A particular example is given in Example 3, with latex bead particles of different colours dried on one side and with latex bead conjugates and colloidal gold conjugates dried on the other side of the device.

Another embodiment of the present invention concerns a double-sided chromatographic device (1), wherein the conjugate pads are not the same (different, not identical, for instance made of different materials) on both sides (side #1 and side #2) of the device (1). This is technically impossible to achieve on single-sided devices. For instance, an embodiment of the invention concerns a device, wherein the membranes of the application zones or subzones (3 and 3') are not the same on both sides of the device (1). According to a preferred embodiment, on one side of the device a membrane (11') of the application zone bears the conjugate(s) while the membrane on the other side (11) overlaps with another membrane (13) bearing the conjugate(s), also referred to as the conjugate membrane.

Yet another aspect of the invention concerns a method to detect and/or quantify at least one analyte or analyte analogue in a test sample by detecting the presence or absence of said at least one analyte or analyte analogue in a sample put in contact with the sheet-like chromatographic device (1) according to the invention.

A particular embodiment of the invention concerns a method as described above wherein the development or not of a signal (for instance a colored signal) at the position of the immobilization of the test or analyte-specific capture reagent (6, 6'), such as a test or analyte-specific antibody, indicates the presence or absence of an analyte or analyte analogue.

Advantageously, the development of a signal (for instance a colored signal) at the position of the immobilization of the control capture reagent (8), such as a migration control antibody, indicates that the sample has migrated on both sides (side #1 and side #2) of the (immuno)chromatographic device (1) according to the invention.

Advantageously, the development of a signal (for instance a colored signal) at the position of the immobilization of the control reagent, such as a control antibody, indicates the correct use and good condition of the sheet-like (immuno)chromatographic device (1) according the invention, the quality of the dried conjugates (7, 7', 9, 9', 12), as well as the completion of the capture reaction such as an immunological reaction.

Advantageously, the development of a signal (for instance a colored signal) at the position of the immobilization of a control capture reagent (migration and possibly reference control reagent), such as a control antibody, is independent of the presence or absence of the analyte or analyte analogue to detect in the sample.

A double-sided sheet-like chromatographic device (1) according to the invention can be used for instance to detect the presence of Influenza A and/or Influenza B with one single strip, side #1 of said strip containing test capture reagents (6) such as antibodies specific for Influenza A and side #2 containing test capture reagents (6') such as antibodies specific for Influenza B.

A double-sided sheet-like chromatographic device (1) according to the invention can further be used to detect the presence of *Giardia lamblia* and/or *Cryptosporium parvum* with one single strip, side #1 of said strip containing test capture reagents (6) such as antibodies specific for *Giardia lamblia* and side #2 containing test capture reagents (6') such as antibodies specific for *Cryptosporium parvum.*

### Brief description of the figures

Figures 1 and 2 give an example of a double-sided chromatographic device (1), according to the invention with on each side of an active membrane supporting polymer (2) a sample application zone (3, 3'), a detection zone (4, 4') comprised of for instance nitrocellulose as active membrane (10, 10') and an absorbent zone (5, 5') comprising absorbent membrane (11, 11'). Migration control (8) and capture reagents (6, 6') are deposited at different levels of the detection zone (4, 4'). Capture reagents may be either analyte specific capture reagents or reference (control) capture reagents. The specific conjugates (7, 7'), migration control conjugates (9, 9') and reference control conjugates (12) preferably comprise either gold particles or latex microspheres as direct label and result in the appearance of control and test signals.

Figure 2 particularly shows a device (1) with different absorbent membranes. Side #1 of the device shown in Figure 2 shows two different membranes in the application zone, the first one is an absorbent membrane (11) that overlaps with a second membrane (13) that is impregnated with the conjugate(s) (see hatched subzone). On side #2 of the same Figure, the conjugates are impregnated into the absorbent membrane (see dotted subzone).

### Detailed description of the invention

The present invention relates in particular to improved chromatographic devices such as improved lateral flow, or dipstick devices and their use in the detection of (multiple) analytes or analyte analogues possibly present in a test sample such as a biological sample. Preferred devices comprise on both sides (side #1 and side #2) of a supporting polymer (2) an application zone or region (3, 3'), a detection zone or region (4, 4') and possibly an absorption zone or region (5, 5') as known in the art. Both detection regions or zones (4, 4') may contain several defined subzones, preferably lines, each dedicated to the detection of one or more particular analytes, a group of analytes or of particular analyte products. There may be included one or more control zones or regions possibly containing several subzones. The devices of the invention may be one-piece sheet-like devices or may be comprised of several parts in contact with each other.

Prior art documents such as EP 0 088 636, EP 0 186 799, EP 0 284 232 and WO 88/08534 are referenced to with respect to the principles of (immuno)chromatographic devices and the reaction between the different compounds such as analyte, conjugate and capture reagent such as an immunoreagent. The disclosure of these documents is herein incorporated in their entirety by reference thereto.

Dipstick devices draw the sample through the membrane perpendicularly, while the sample is permitted to flow laterally from an application zone or region to a reaction zone or region on the membrane surface with the lateral flow system.

The devices (1) according to the invention are composed of porous polymeric substances that preferably are laminated on both (opposite) sides (side #1 and side #2) of a rigid or semi-rigid polymer (2) to provide mechanical strength, which makes the devices (1) of the invention easy to handle. The porosity of the polymeric substances should be such that movement of a fluid and its components from the bottom to the top of the stick (1), moving along rehydrated conjugate is possible without any hindrance. These characteristics are allowed by the hydrophilic properties of these polymers. Examples of suitable polymers are cellulose, nitrocellulose, cellulose acetate, glass fibres, nylon, acrylic copolymer/nylon, polyethersulfone, polyethylene and polyester.

The term analytes or analyte analogues, as used herein, relates to molecules to be detected in a biological samples. Examples of such molecules include proteins, peptides, haptens, polysaccharides, lipopolysaccharides, nucleic acids, viral particles, parts of micro-organisms such as bacteria, viruses, protozoans and parasites, or chemical compounds of any origin. The devices (1) according to the invention are in particular useful for the detection of harmful microorganisms or compounds thereof in biological samples, including but not limited to the detection of *Cryptosporidium parvum* oocysts, *Giardia lamblia* cysts, RSV (Respiratory Syncytial Virus), Respiratory Adenovirus, Influenza-A and -B viruses, Rotavirus, Adenovirus type 40/41 and Adenovirus groups. Advantageously, a device can be designed that allows detection of several such analytes or analyte analogues via one single test (1) and/or allows detection of more than one harmful compound produced by a given analyte such as *E.coli* shiga-like toxins I and II, and/or allows detection of multiple serological compounds such as IgG, IgA and IgM raised after an infection by a pathogen.

The test sample, preferably a liquid test sample, suspected to contain an analyte or analyte analogue, may be derived from any biological sample, including but not limited to culture supernatants, nasopharyngeal secretions, stool specimens, serum,.... Samples such as for instance stool specimens are prior to application preferably suspended in a solution that allows migration of the liquid through the device (1).

Specific labeled reagents (7, 7') that are specific for the analytes or analyte analogues serve to detect and/or quantify analytes or analyte analogues possibly present in a sample. The specific labeled reagents (conjugates) (7, 7') will individually form a complex with individual analytes or analyte analogues, which complexes are then captured by analyte-specific reagents (6, 6'). A labeled reagent may be used to react finally with a control reagent adsorbed preferably onto the second porous region or zone. The capture reagents (6, 6', 8) may be polyclonal or monoclonal antibodies or any hypervariable antibody fragment known in the art. Preferably monoclonal antibodies or hypervariable fragments thereof are used. These capture reagents (6, 6', 8) may be produced via genetic engineering.

Labeled reagents or detection agents (7, 7', 9, 9', 12) are immobilized (impregnated) on an inert material that can be glass fibres or polyester or any other material physically and chemically inert and with sufficient porosity and wettability to allow particle movement and to allow labeled reagents to rehydrate easily and completely when liquid sample reaches them. When the liquid sample is in contact with these rehydrated detection agents, individual analytes will form a complex with their specific labeled reagent and these complexes will react with their specific immunoreagents adsorbed on the detection region or zone while the labeled control agent will move freely up to the control reagent adsorbed onto the control region or zone to react therewith.

Various detection systems are known in the art. Colored or visible (direct) particulate labels known in the art include particles made of latex polymers, metallic colloids such as gold, carbon, liposomes, silver, copper, ... which can be conjugated to the binding reagent that normally reacts with the analytes to be detected. Quantification and/or semi-quantification are possible.

The present invention relates to a method for rapid and specific identification of several pathogens from biological samples, or laboratory samples with the same device, a double-sided sheet-like chromatographic device according to the invention (1).

In a preferred embodiment of the invention specific conjugates (7, 7') comprise visible (direct) labels to which reagents specific to analytes or analytes analogues are bound (conjugated therewith). The complex formed between the analytes or analyte analogues and their specific conjugates (7, 7') will move onto the membrane of the detection zone (preferably nitrocellulose) (4, 4') and reach specific analytes or analytes analogues reagents (6, 6') coated thereon. The reaction between the complexes and the specific reagents (6, 6') to the analytes or analyte analogues will be visualized since the particles will accumulate and generate a visible signal. This signal allows the user to identify specifically which analyte or analyte analogue is present in the analyzed sample and preferably also to quantify or semi-quantify (possibly via a reference line) the amount thereof present in the test sample.

Below, more details are provided with respect to general aspects and preferred compositions and build-up of the particular the chromatographic devices according to the present invention.

To conduct the chromatographic assay, the device (1) according to an embodiment of the invention is preferably divided into three zones or regions including an application zone (3, 3'), a detection zone (4, 4') and an absorbent zone (5, 5') located on both sides of the device.

In a preferred embodiment, the membranes of the application zones or regions (3, 3') are made of glass fibres or cellulose with conjugate pads made of polyester, the membranes of the detection zones or regions (4, 4') are made of nitrocellulose, and the membranes of the absorbent zones or regions (5, 5') are made of cellulose. In particular embodiments, application zones or regions (3, 3') and conjugate pads can be made of the same matter or material. The conjugates (7, 7', 9, 9', 12) can, however, also be impregnated directly onto the application zones.

In another preferred embodiment both application zones or regions (3, 3') are made of a rigid polymer on which adheres an absorbent membrane made of glass fibres to absorb and conduct the sample liquid to the detection zones (4, 4') by covering them. The glass fibres may further cover a conjugate membrane (13 and 13') made of polyester that contains the conjugate (7, 7', 9, 9', 12) under a dry form. Also these membranes should have such characteristics that the conjugates will be easily hydrated by the sample liquid to allow a complete removal of the hydrated conjugates and specific reaction of the conjugate reagents with their specific analytes.

The conjugate membranes (13 and 13') can be fully or partially covered by the absorbent membranes (11, 11'). Both absorbent membranes and conjugate membranes could be made of the same matter or material. In this case, the conjugates (7, 7', 9, 9', 12) could be directly sprayed onto the polymer that is also used to absorb the sample liquid. Both possibilities are indicated in Figure 2.

The conjugate pads are impregnated with particles that are coated with some compounds that could include proteins, peptides, haptens, polysaccharides, lipopolysaccharides, nucleic acids to form a an analyte-specific conjugate (7, 7'). These compounds will react somewhere specifically with analytes or analyte analogues that could be present into the sample(s) to be analyzed. Examples of particles include colloidal gold particles; colloidal sulphur particles; colloidal selenium particles; colloidal barium sulfate particles; colloidal iron sulfate particles; metal iodate particles; silver halide particles; silica particles; colloidal metal (hydrous) oxide particles; colloidal metal sulfide particles; colloidal lead selenide particles; colloidal cadmium selenide particles; colloidal metal phosphate particles; colloidal metal ferrite particles; any of the above-mentioned colloidal particles coated with organic or inorganic layers; protein or peptide molecules; liposomes; colored microparticles or organic polymer latex particles.

In a preferred embodiment, particles are colloidal gold particles or latex microspheres. Colloidal gold particles could be of about 5 to about 60 nm of diameter. Preferably, particles of about 20 nm or about 40 nm diameter are used. Polystyrene latex beads that have been activated with several chemical functions such as carboxyl one, amine one, hydroxyl one and sulfhydril one could be used. In one preferred embodiment, non-activated latex particles are used. Preferably, microspheres of about 150 nm to about 350 nm diameter are used.

In order to perform multicolor detections, different colored microspheres are used (e.g red for analyte A and blue for analyte B).

Analyte-specific particles to be used in the double-sided sheet-like chromatographic devices (1) of the invention are coated with compounds that specifically bind with the analyte or analyte analogue to be detected.

The detection zone or region (4, 4') of the double-sided sheet-like chromatographic devices (1) according to the invention could be made of cellulose, nitrocellulose, cellulose acetate, glass fibres, nylon, acrylic copolymer/nylon, polyethersulfone, polyethylene and polyester but preferably is made of nitrocellulose from Advanced Microdevices Pvt, Ltd. Membranes from other supplier (Schleicher & Schuell or Millipore or Porex or Pall) can also be used.

Coating preferably is performed by diluting the reagents (6, 6', 8) in an appropriate buffer and by distributing them onto the membrane, preferably nitrocellulose (4, 4'), with a contact system (e.g. IsoFlow from Imagen Technology). Speed distribution could vary from about 50 mm to about 10 mm/sec but is preferably fixed to about 40 mm/sec or even better at about 30 mm/sec. Volume of material distributed varies from about 0.5 to about 3 µl/cm, preferably from about 0.7 to about 2 µl/cm and more precisely from about 1 to about 2 µl/cm.

Reagent concentration varies from about 0.1 to about 10 mg/ml and preferably is about 0.15 to 2 mg/ml. In a preferred embodiment of the invention, the buffer used for this coating consisted of a saline solution (NaCl) buffered with phosphate at about pH 7.2.

In an embodiment of the invention, the double-sided sheet-like chromatographic devices (1) of the invention include absorbent zones or regions (5, 5') that aspirates solution that has been transported to the end of the nitrocellulose (4, 4'). Examples of substances include cellulose and glass fibres. Cellulose (MDI) or glass fiber (Schleicher & Schuell) have been preferably used.

The double-sided sheet-like chromatographic devices (1) of the invention, preferably also include control subzones (amongst other internal control and/or migration control) preferably containing one or more control lines. The migration control conjugate should not react with the specific conjugates (7, 7'), nor with the analyte itself, nor with anything that could be present in the sample to be analyzed. Preferably the migration control line is built in such a way that its intensity is always the same and does not depend on the specific signal and its intensity. Coating of the control reagent is as described above. The internal migration capture conjugate is either mixed in the conjugate pad with the specific conjugates (7, 7'), either impregnated alone for instance on the conjugate membrane located on the reverse side of the device. Liquid sample migrates to the conjugate membranes and rehydrates the conjugates, i.e. the control conjugate and specific analyte or analyte analogue conjugates (7, 7'). If related analytes or analyte analogues are present, several complexes will be formed. Since the flow progresses through the preferably nitrocellulose membranes (10, 10'), the said complexes will give rise to visible (e.g. red, bleu, green,...) lines or subzones in case of positive reactions on both sides (side #1 and side #2) of the device (1), while the control conjugate proceeds on one's way to reach and react with its coated reagent leading to a visible line also either on both (opposite) sides (side #1 and side #2) of the said device or only on one side (side #1 or side #2) of the device (1), for instance if it is used as quantification reference. The control signal indicates amongst others that the test has been properly performed, appearing also in the absence of a specific reaction. The control signal may further serve as a quantitative reference. Specific and control signals can be of the same or a different color. Size of particles of the control conjugates and of the specific conjugates can be the same or can be different.

In a particular embodiment according to this invention, both (control and specific) conjugates (7, 7', 9, 9', 12), preferably gold conjugates, are impregnated into a solid inert membrane that could be polyester or nylon. Polyester is preferred. The polyester membranes used here have a size of 27 x 260 mm and are from Advanced Microdevices Pvt, Ltd (India). The membranes are impregnated with the preferably gold conjugates after a dilution step in a specific buffer to provide an optimal rehydratation with the liquid sample when the test is running. AccuFlow G membranes from Schleicher & Schuell are also useful for this purpose and give the advantage that the conjugates (7, 7', 9, 9', 12) are directly sprayed onto the absorbent membrane (see for instance Figure 2 - side #2 thereof).

When using the polyester membranes from Advanced Microdevices Pvt, the membranes are impregnated by dipping into an appropriate vial with a finite volume that is 1,6 ml but that could be reduced to 1,3 ml depending on the impregnation system used. Membranes are let to dry at room temperature overnight. They are then dried in an oven at about 55 °C for about 20 minutes. After drying, those membranes are stored in specific boxes with desiccants under a maximum of 10 % of relative humidity. Membranes are cut into about 5 mm width pieces and sticked onto both adhesive parts of the laminates. Location of these membranes is important to reach the maximum detectability expected for specific purposes. Absorbent papers made of glass fibres, or any other absorbent matter, are then sticked onto both adhesive parts of the strip provided they cover tightly the polyester membranes to allow the liquid to rehydrate the conjugates and let them react with the analytes or analyte analogues present in the sample.

When AccuFlow G membranes are used, the conjugates (7, 7', 9, 9', 12) are sprayed with the IsoFlow Atomizing Nozzle system from Imagen Technology. In this case the conjugates (7, 7', 9, 9', 12) are sprayed at a speed of 50 mm/sec for quantities sprayed ranging from 0.8 µL/mm to 1.67 µL/mm with a pressure ranging from 1 to 20 psi.

Some tests require that a quantification is performed in order to know whether the concentration of for instance the antigen or whether the serological response to for instance an antigen to be detected is higher or lower than a defined cut-off level. This can be done by comparing the intensity of a test line (specific line) located on one side of the device (side #1 or side #2) in an (immuno)chromatographic test to that of one or several reference line(s) of constant or progressive intensities located on the other side of the device. The reference scale that is obtained as such consists of several lines of different intensities between them, but constant and reproducible for each of them. Each conjugate (7, 7', 9, 9', 12) is hereby preferably dried at a predefined concentration to obtain a constant intensity when the conjugate accumulates on the corresponding coated antibody upon migration of a sample in the double-sided sheet-like chromatographic device (1) of the invention. The intensity of the test line signal located on one side of the device (side #1 or side #2) will be proportional to the concentration of for instance the antigen, at least in a desired predefine range of concentrations including the cut-off level.

The present invention is further illustrated by the following examples, which are not intended to be limiting in any way.

### EXAMPLES

### Materials and methods.

### Example 1: Detection of Influenza viruses A and B, Respiratory Adenovirus and Respiratory Syncytial virus.

### Preparation of colloidal gold particles

Colloidal gold particles of about 20nm were prepared by reduction of tetrachloroauric acid with sodium citrate. Two hundred ml of ultrapure water containing about 60 mg of HAuCl₄.3H₂O were heated to boiling and about 5 ml of a 4% dihydrate sodium citrate solution were added. Boiling was continued for a few minutes, until a dark red solution was obtained. The solution was let to equilibrate at room temperature before use. The OD₅₂₀ₙₘ was measured to evaluate particles concentration in the solution.

Alternatively, colloidal gold particles of about 40nm were purchased from a commercial source (Diagam).

### Coupling of antibodies to colloidal gold particles

Coupling antibodies to colloidal gold particles is well known in the art. In this example, mouse monoclonal antibodies directed against Influenza A virus, Influenza B virus, Adenovirus or Respiratory Syncytial virus were used. Purified antibodies were reacted with a colloidal gold particles suspension that had been buffered with a potassium carbonate solution to obtain the desired pH. This pH is predetermined and may be different for each antibody. The dilution of the purified antibody to be used in the coupling process was defined in a preliminary experiment.

In this preliminary experiment, increasing dilutions of one antibody were reacted for three minutes with the buffered colloidal gold particles and then sodium chloride was added to reach about 1% final concentration. Absorbance at 630nm was recorded. The highest dilution of the antibody at which the absorbance was equal or similar to the absorbance obtained with the lower dilution of the antibody was chosen as the reference dilution for the coupling of the antibody to the colloidal gold particles.

For the coupling in itself, the antibody at the chosen dilution and the buffered colloidal gold particles were reacted for three minutes. This so-called conjugate was subsequently saturated and washed several times by centrifugation and resuspension in a washing buffer to remove any unconjugated antibodies and finally resuspended in a conservation buffer.

### The immunochromatographic device

The double-sided sheet-like immunochromatographic device (1) of the present example consists of a plastic backing solid support (MDI) (2) with thereupon a sample application region (3, 3') consisting of AccuflowG (Shleicher & Schuell) on both sides, a detection region made of nitrocellulose (MDI) on one side (side #1) (4) and of Porex Lateral-Flo Membrane (Porex) on the other side (side #2) (4') and an adsorption region (5, 5') made of cellulose (MDI) on both sides. The Strip exemplified here is a Strip wherein the active membranes (10, 10') of the detection zone (4, 4') of both sides are different, in this case are made of different materials.

One side, side #1, is dedicated to the detection of the Influenza A virus and the Influenza B virus. The other side, side #2, is dedicated to the detection of the Adenovirus and the Respiratory Syncytial virus. This device is called hereinafter RespiVirus double-sided Strip.

On side #1, the nitrocellulose membrane is sensitized with three reagents. The first reagent is a mouse monoclonal antibody directed against the Influenza A virus and is deposited in the lower subregion of the detection region. This is defined as the "A test line". The second reagent is a mouse monoclonal antibody directed against the Influenza B virus and is deposited in the middle subregion of the detection region. This is defined as the "B test line". The third reagent is a goat polyclonal antibody directed against mouse monoclonal antibodies, and will react with both the anti-Influenza A virus and the anti-Influenza B virus antibodies used for coupling to the colloidal gold particles, and is deposited in the upper subregion of the detection region. This is defined as the "Side #1 migration control line". The Influenza A virus specific and the Influenza B virus specific conjugates are deposited in the upper subregion of the application membrane (AccuflowG) of the application region of this side #1.

On side #2, the detection membrane is made of the Porex Lateral-Flo K membrane sensitized with three reagents. The first reagent is a mouse monoclonal antibody directed against the Adenovirus and is deposited in the lower subregion of the detection region. This is defined as the "Ad test line". The second reagent is a mouse monoclonal antibody directed against the Respiratory Syncytial virus and is deposited in the middle subregion of the detection region. This is defined as the "RSV test line". The third reagent is a goat polyclonal antibody directed against mouse monoclonal antibodies, and will react with both the anti-Adenovirus and the anti-Respiratory Syncytial virus antibodies used for coupling to the colloidal gold particles, and is deposited in the upper subregion of the detection region. This is defined as the "Side #2 migration control line". The Adenovirus and Respiratory Syncytial virus specific conjugates are deposited in the upper subregion of the application membrane (AccuflowG) of the application region of this side #2.

### Carrying out of the test.

The test with the RespiVirus double-sided Strip is carried out as usually performed with single-side immunochromatographic ("dipstick") tests.

Samples suspected to contain Influenza A or Influenza B virus, or Adenovirus or Respiratory Syncytial virus are diluted in a sample buffer and the RespiVirus double-sided Strip is dipped into these solutions.

The Influenza A test (on side #1) was shown to be specific: no A test line appears with a solution containing no virus, or Influenza B virus, or Adenovirus, or Respiratory Syncytial virus. The A test line appears with a sample containing Influenza A virus, and the intensity decreases with increasing dilutions of the virus.

The Influenza B test (on side #1) was shown to be specific: no B test line appears with a solution containing no virus, or Influenza A virus, or Adenovirus, or Respiratory Syncytial virus. The B test line appears with a sample containing Influenza B virus, and the intensity decreases with increasing dilutions of the virus.

The Adenovirus test (on side #2) was shown to be specific: no Ad test line appears with a solution containing no virus, or Influenza A or B viruses, or Respiratory Syncytial virus. The Ad test line appears with a sample containing Adenovirus, and the intensity decreases with increasing dilutions of this virus.

The Respiratory Syncytial virus test (on side #2) was shown to be specific: no RSV test line appears with a solution containing no virus, or Influenza A or B viruses, or Adenovirus. The RSV test line appears with a sample containing Respiratory Syncytial virus, and the intensity decreases with increasing dilutions of this virus.

In all cases, both Side #1 and Side #2 migration control lines appear, showing that the sample has migrated on both (opposite) sides (side #1 and side #2) of the RespiVirus double-sided Strip.

### Example 2: Detection of Giardia lamblia and Cryptosporidium parvu.

### Preparation of colloidal gold particles

This was performed as described in the first example.

### Coupling of antibodies to colloidal gold particles

Coupling of the antibodies to the colloidal gold particles was performed essentially as described in the first example. These couplings were performed with mouse monoclonal and rabbit polyclonal antibodies directed against *Giardia lamblia* and a mouse monoclonal antibody directed against *Cryptosporidium parvum.*

### The immunochromatographic device

The present Strip is an example of a Strip with on each side nitrocellulose as active membrane, the porosity of the membrane on side #1 being different though from that on side #2. Also the membranes of the application zone differ, id est are not the same on both sides of the device (1).

The double-sided sheet-like immunochromatographic device (1) of the present example consists of a plastic backing solid support (MDI) (2) with thereupon application, detection and absorption regions on both sides.

On one side (side #1) the application region (3) consists of a polyester membrane (MDI) covered with a cellulose membrane (MDI), the detection region (4) is made of nitrocellulose (MDI) that has a 8*µ*m porosity and the absorption region (5) is made of cellulose (MDI).

On the other (opposite) side (side #2), the application region (3') consists of AccuflowG (Schleicher & Schuell), the detection region (4') is made of nitrocellulose (MDI) that has a 15*µ*m porosity and the absorption region (5') is made of cellulose (MDI).

Side #1 is dedicated to the detection of *Cryptosporidium parvum.* Side #2 is dedicated to the detection of *Giardia lamblia.*

On side #1, the 8*µ*m nitrocellulose membrane is sensitized with two reagents. The first reagent is a mouse monoclonal antibody directed against *Cryptosporidium parvum* and is deposited in the lower subregion of the detection region. This is defined as the "C test line". The second reagent is deglycosylated recombinant ProteinA and will react with the *anti-Cryptosporidium parvum* antibody used for coupling to the colloidal gold particles, and is deposited in the upper subregion of the detection region. This is defined as the "Side #1 migration control line". The *Cryptosporidium parvum* specific conjugate is impregnated in the polyester membrane (MDI) present in the upper subregion of the application region of this side #1.

On side #2, the 15 *µ*m nitrocellulose membrane is sensitized with two reagents. The first reagent is a mouse monoclonal antibody directed against *Giardia lamblia* and is deposited in the lower subregion of the detection region. This is defined as the "G test line". The second reagent is deglycosylated recombinant ProteinA and will react with the anti- *Giardia lamblia* antibodies used for coupling to the colloidal gold particles, and is deposited in the upper subregion of the detection region. This is defined as the "Side #2 migration control line". The *Giardia lamblia* specific conjugates are deposited in the upper subregion of the application membrane (AccuflowG) of the application region of this side #2.

### Carrying out of the test.

The test with the *Giardia lamblia*/*Cryptosporidium parvum* double-sided immunochromatographic device (1) of the invention is carried out similarly as described in the first example.

Samples containing either *Giardia lamblia* or *Cryptosporidium parvum* are diluted in a sample buffer and the *Giardia lamblia*/*Cryptosporidium parvum* double-sided immunochromatographic device (1) of the invention is dipped into these solutions.

The *Cryptosporidium parvum* test (side #1) was shown to be specific: no C test line appears with a solution containing no pathogen or *Giardia lamblia* only. The C test line appears with a sample containing *Cryptosporidium parvum,* and the intensity decreases with increasing dilutions.of the sample.

Similarly, the *Giardia lamblia* test (on side #2) was shown to be specific: no G test line appears with a solution containing no pathogen or *Cryptosporidium parvum* only. The G test line appears with a sample containing *Giardia lamblia*, and the intensity decreases with increasing dilutions of the sample.

In all cases, both side #1 and side #2 migration control lines appear, showing that the sample has migrated on both sides (side #1 and side #2) of the immunochromatographic device (1).

### Example 3: Detection of Rotavirus, Adenovirus group (group A to F) and Enteric Adenovirus 40/41 (group F, strains 40 & 41).

### Preparation of colloidal gold particles

This was performed as described in the first example.

### Coupling of antibodies to colloidal gold particles

Coupling of the antibodies to the colloidal gold particles was performed essentially as described in the first example. These couplings were performed with a mouse monoclonal antibody directed against Adenovirus (group).

### Coupling of antibodies to microsphere ("latex") particles

Microsphere ("latex") particles were obtained from a commercial source (Estapor). These microspheres were either without any specific functional groups on their surface (hereinafter called latex beads) or with amine groups on their surface (hereinafter called NH2-latex beads). Coupling of the reagent to these microsphere particles was performed essentially following the protocol provided by the manufacturer. Couplings with the latex beads were performed with a mouse monoclonal antibody directed against Enteric Adenoviruses (40/41) (red latex beads) or with neutralite avidin (green latex beads). Couplings with the blue NH2-latex beads were performed with a mouse monoclonal antibody directed against Rotavirus or with a peptide called 7a whose sequence is DQFGNLGVV (SEQ ID NO: 1).

### The immunochromatographic device

In the present Strip, the conjugates dried on one side of the Strip differ from conjugates dried on the other side of this Strip. In the present strips, a mix of different labels is present on each side.

The double-sided sheet-like immunochromatographic device (1) of the present example consists of a plastic backing solid support (MDI) (2) with thereupon a sample application region (3, 3') consisting of Glass33 (Shleicher & Schuell) on both sides, a detection region (4, 4') made of nitrocellulose (MDI) on both sides and an adsorption region (5, 5') made of cellulose (MDI) on both sides.

One side, side #1, is dedicated to the detection of the Rotavirus and the Enteric adenovirus (40/41). The other (opposite) side, side #2, is dedicated to the detection of the Adenovirus (group). This device is called hereinafter EntericVirus double-sided Strip.

On side #1, in the following example, the nitrocellulose membrane is sensitized with three reagents. The first reagent is a guinea pig polyclonal antibody directed against Rotavirus and is deposited in the lower subregion of the detection region. This is defined as the "Rota test line". The second reagent is a monoclonal antibody directed against Adenovirus and is deposited in the middle subregion of the detection region. This is defined as the "Ad40/41 test line". The third reagent is biotinylated albumin and will react with the neutralite avidin used for coupling to the green latex beads, and is deposited in the upper subregion of the detection region. This is defined as the "Side #1 migration control line". Three conjugates are used on this side #1 of the EntericVirus double-sided Strip: the monoclonal antibody directed against rotavirus conjugated to blue NH2-latex, the monoclonal antibody directed against the enteric adenoviruses (40/41) conjugated to the red latex beads and the neutralite avidin conjugated to green latex beads. The mix of these three conjugates is deposited in the upper subregion of the glass fiber application membrane (Glass 33 from Schleicher & Schuell) of the application region of this side #1 of the EntericVirus double-sided Strip.

On side #2 of the EntericVirus double-sided Strip, the nitrocellulose membrane is sensitized with two reagents. The first reagent is a mouse monoclonal antibody directed against Adenovirus (group) and is deposited in the lower subregion of the detection region. This is defined as the "Ad test line". The second reagent is a mouse monoclonal antibody directed against peptide 7a, and is deposited in the upper subregion of the detection region. This is defined as the "Side #2 migration control line". The Adenovirus (group) colloidal gold conjugate is mixed with the peptide 7a-NH2-latex beads (blue) conjugate and they are deposited in the upper subregion of the application membrane (Glass33) of the application region of this side #2 of the EntericVirus double-sided Strip.

### Carrying out of the test.

The test with the EntericVirus double-sided Strip of the invention is carried out similarly as described in the first example.

Samples containing either Rotavirus or Enteric Adenoviruses (40/41) or Adenovirus (group) are diluted in a sample buffer and the EntericVirus double-sided Strip of the invention is dipped into these solutions.

The presence of Rotavirus will be detected by the appearance of a blue line in the lower subregion of detection region of side #1 (Rota test line), and the presence of enteric adenoviruses (40/41) will be detected by the appearance of a red line in the middle subregion of detection region of side #1 (Ad 40/41 test line). The migration of the sample on this side #1 of the EntericVirus double-sided Strip will bring the neutralite avidin-green latex beads conjugate to the side #1 migration control line where neutralite avidin will react with the coated biotynylated albumin giving rise to a green migration control line.

The presence of Adenovirus (group) will be detected by the appearance of a red/purple line in the lower subregion of detection region of side #2. The migration of the sample on this side #2 of the EntericVirus double-sided Strip will bring the peptide 7a-blue NH2-latex beads conjugate to the side #2 migration control line where peptide 7a will react with coated anti-peptide 7a monoclonal antibody giving rise to a blue migration control line.

In all cases, both side #1 and side #2 migration control lines appear, showing that the sample has migrated on both sides (side #1 and side #2) of the immunochromatographic device (1).

### Example 4: Quantitative or semi-quantitative detection of RSV virus.

### Preparation of colloidal gold particles

This was performed as described in the first example.

### Coupling of antibodies to colloidal gold particles

Coupling of the antibodies to the colloidal gold particles was performed essentially as described in the first example. These couplings were performed with mouse monoclonals directed against the RSV and three mouse monoclonal antibodies directed against three different peptides. These peptides were supplied grafted on a carrier protein like bovine serum albumin (BSA).

### The immunochromatographic device

The present Strip is an example of a Strip with a test side and a control side, the control side bearin at least one migration control capture reagent and migration control conjugate, and and least two reference capture reagents plus their relative conjugates.

The double-sided sheet-like immunochromatographic device (1) of the present example consists of a plastic backing solid support (MDI) (2) with thereupon application, detection and absorption regions on both sides.

On one side, hereinafter called the #1 side, the application region (3) consists of AccuflowG (Schleicher & Schuell), the detection region (4) is made of nitrocellulose (MDI) that has preferably but not limited to a 10 *µ*m porosity and the absorption region (5) is made of cellulose (MDI) .

On the other (opposite) side, hereinafter called the #2 side, the application region (3') consists of AccuflowG (Schleicher & Schuell), the detection region (4') is made of nitrocellulose (MDI) that has preferably but not limited to 10 *µ*m porosity and the absorption region (5') is made of cellulose (MDI) .

One side, called the #2 side, is dedicated to the detection of RSV virus that could be present in the analysed sample. The other side, called the #1 side, is dedicated to the semi-quantification of the detected RSV.

On the #2 side, the nitrocellulose membrane is sensitized with two reagents. The first reagent is a mouse monoclonal antibody directed against the RSV protein F and is deposited in the lower subregion of the detection region. This is defined as the "T test line". The second reagent is an anti-mouse IgG antibody and will react with the anti-RSV antibody used for coupling to the colloidal gold particles, and is deposited in the upper subregion of the detection region. This is defined as the "C migration control line". The RSV specific conjugate is made of an anti-RSV protein F antibody and is impregnated in the AccuflowG (Schleicher & Schuell), present in the application region of this #2 side.

### Side #1 - version #1

On the #1 side, the nitrocellulose membrane is sensitized with three or four reagents. The first reagent is an anti-mouse IgG antibody directed against the anti-peptide antibodies coupled to gold particles used and is deposited in the upper part subregion of the detection region. This is defined as the "C migration control line" or "C line" as well as for the side #2. Coating conditions are very similar to those described in the above text. The three other reagents are three peptides carried by BSA and coated at three different locations of the detection region, but always below the "C line". Each of these peptides will react with its specific mouse monoclonal antibody coupled to gold particles. The three peptides used in the present example are:
- PPSFCPNFIPCTDGL (SEQ ID NO: 2)
- DQFGNLGW (SEQ ID NO: 1)
- ADGARLKGGVGAAGA (SEQ ID NO: 3)

This is defined as the "R Reference lines" or "R lines".

Coating conditions are very similar to those referenced in the above text.

The three anti-peptide specific conjugates are deposited in the upper subregion of the application membrane (AccuflowG) of the application region of this #1 side in such quantities that they will develop signals with different intensities. In the present example, the first line relates to RSV quantities expressed in CFU/mL such as 3.7 10⁵ CFU/mL, the second line located above the first line relates to RSV quantities such as 7.4 10⁵ CFU/mL and the third line located above the second one but below the "C line" relates to RSV quantities such as 1.4 10⁶ CFU/mL.

### Side #1 - Version #2

On the #1 side, the nitrocellulose membrane is sensitized with two reagents. The first reagent is an anti-mouse antibody directed against the anti-peptide antibody coupled to gold particles used and is deposited in the upper part subregion of the detection region. This is defined as the "C migration control line" or "C line". Coating conditions are very similar to those described in the above text.

In this example, a peptide is coated at different concentrations ranging from 0.1 mg/mL to 1 mg/mL at different location of the detection region but always below the "C line". This peptide will react with its specific mouse monoclonal antibody coupled to gold particles. The peptide used in the present example is:

### - PPSFCPNFIPCTDGL (SEQ ID NO: 2)

This is defined as the "R Reference lines" or "R lines".

Coating conditions are very similar to those referenced in the above text.

The anti-peptide specific conjugate is deposited in the upper subregion of the application membrane (AccuflowG) of the application region of this #1 side in such quantities that it will develop signals with different intensities depending on the quantities of peptide coated. In the present example, the first line relates to RSV quantities expressed in CFU/mL such as 3.7 10⁵ CFU/mL, the second line located above the first line relates to RSV quantities such as 7.4 10⁵ CFU/mL and the third line located above the second one but below the "C line" relates to RSV quantities such as 1.4 10⁶ CFU/mL. The first peptide line is coated in such conditions that all the coated peptides will react with the conjugate allowing the remaining conjugate to migrate to second line for reacting with all the peptides thereon coated and allowing the remaining conjugate to migrate to the third line for the same purpose. All the coated peptides are so saturated with the conjugate showing signal of increasing intensity from the first line to the third one. Enough conjugate remains to react with the anti-mouse reagent to generate the "C line".

### Carrying out of the test.

The present test aimed at the semi-quantitative detection of the RSV virus with the double-sided immunochromatographic device (1) of the invention is carried out similarly as described in the first example.

Samples containing RSV virus are diluted in an extraction buffer and incubated so for 10 minutes at room temperature. The semi-quantitative RSV double-sided immunochromatographic device (1) of the invention is then dipped into these solutions.

The RSV test (side #2) was shown to be specific: The "T test" line appears with a sample containing RSV, and the intensity decreases with increasing dilutions of the sample.

Similarly, the "Reference lines" (on side #1) appear with increasing intensities by passing from the first line to the third one.
By comparing signal intensities on both sides, it will be possible to determine the RSV concentration in the sample.

In all cases, both C migration control lines appear, showing that the liquid has migrated on both sides (side #1 and side #2) of the immunochromatographic device (1).

## Claims

1. A double-sided sheet-like chromatographic device (1), in particular a dipstick or lateral flow device, with on both sides of a rigid solid support (2)
- an application zone or region (3, 3') optionally with conjugate pad,
- a detection zone or region (4, 4') possibly with a control subzone or subregion, and
- optionally an absorbent zone or region (5, 5');
wherein the detection zone or region of both sides (4, 4') comprises at least one, preferably several capture reagents (6, 6') specifically recognizing analytes or analyte analogues to be detected in the assay sample; and
wherein the application zone of both sides (3, 3') comprises at least one, preferably several analyte specific conjugates (7, 7') with direct or indirect label which allow detection of said analytes or analyte analogues,
said device (1) preferably being an immunochromatographic device and
preferably bearing a control test line with a control capture reagent (8) present on at least one side of the device (1).

2. The device according to any of the preceding claims, wherein the detection region (4, 4') of both sides of the device (1) is sensitized with a test antibody and with a control capture reagent, wherein the test antibodies are directed against the analyte to be detected in the sample, and wherein the control capture reagent is directed either against an anti-analyte antibody that is coupled to a direct label to form an analyte specific conjugate (7, 7'), either against a specific conjugate non relevant to the analytes or analyte analogues to be detected.

3. A double-sided sheet-like chromatographic device (1), in particular a dipstick or lateral flow device, with on both sides of a rigid solid support (2)
- an application zone or region (3, 3') optionally with conjugate pad,
- a detection zone or region (4, 4') possibly with a control subzone or subregion,
- and optionally an absorbent zone or region (5, 5');
wherein side #2 of said device is a test side, the detection zone (4') of which comprises several capture reagents (6') specifically recognizing analytes or analyte analogues to be detected in the assay sample; and the application zone (3') of which comprises several analyte specific conjugates (7') with direct or indirect label which allow detection of said analytes or analyte analogues; and
wherein side #1 of said device (1) is a control side, the detection zone (4) of which comprises at least one migration control capture reagent (8) specific for the migration control conjugate (9) with direct or indirect label comprised in the application zone (3) of said side #1, with the proviso that the migration control on the test side of such a device (1) is not comprised of an oligonucleotide deposited at the upper part of a detection region (4).

4. A double-sided sheet-like chromatographic device (1), in particular a dipstick or lateral flow device, with on both sides of a rigid solid support (2)
- an application zone or region (3, 3') optionally with conjugate pad,
- a detection zone or region (4, 4') possibly with a control subzone or subregion,
- and optionally an absorbent zone or region (5, 5');
wherein side #2 of said device is a test side, the detection zone (4') of which comprises several capture reagents (6') specifically recognizing analytes or analyte analogues to be detected in the assay sample; and the application zone (3') of which comprises several analyte specific conjugates (7') with direct or indirect label which allow detection of said analytes or analyte analogues; and
wherein side #1 of said device (1) is a control side, the detection zone (4) of which comprises at least one migration control capture reagent (8) specific for the migration control conjugate (9) with direct or indirect label comprised in the application zone (3) of said side #1,
said device (1) being an immunochromatographic device.

5. A double-sided sheet-like chromatographic device (1), in particular a dipstick or lateral flow device, with on both sides of a rigid solid support (2)
- an application zone or region (3, 3') optionally with conjugate pad,
- a detection zone or region (4, 4') possibly with a control subzone or subregion,
- and optionally an absorbent zone or region (5, 5');
wherein side #2 of said device is a test side, the detection zone (4') of which comprises one capture reagent (6') specifically recognizing one analyte or analyte analogue to be detected in the assay sample; and the application zone (3') of which comprises one specific conjugate (7') with direct or indirect label which allow detection of said analyte or analyte analogue; and
wherein side #1 of said device (1) is a control side, the detection zone (4) of which comprises at least
- one migration control capture reagent (8) specific for the migration control conjugate (9) with direct or indirect label comprised in the application zone (3) of said side #1, said device (1) being an immunochromatographic device, and
- at least two reference capture reagents (6) specific for their relative conjugates (12) with direct or indirect label comprised in the application zone (3) of said side #1, said device being an immunochromatographic device (1),
wherein these reference reagents will give rise to signals of different intensity to be compared to the signal of the specific analyte or analyte analogue detected on side #2 of said device (1) for quantification purposes.

6. The device according to any of the preceding claims, wherein the porosity of the active membranes (10 and 10') of the detection zone (4 and 4') of both sides is different.

7. The device according to any of the preceding claims, wherein the active membranes (10 and 10') of the detection zone (4 and 4') of both sides are made of different materials.

8. The device according to any of the preceding claims, wherein the conjugates (7, 9, 12) dried on one side of the device (1) bear a label that is different from the label of conjugates (7', 9') dried on the other side of said same device (1).

9. The device according to any of the preceding claims, wherein a mix of conjugates (7, 9, 12) with different labels is dried on at least one side of the device (1), possibly on both sides of said device (1).

10. The device according to any of the preceding claims, wherein the membranes of the application zones or subzones (3 and 3') are not the same on both sides of the device (1).

11. The device according to claim 10, wherein on one side of the device the membrane of the application zone bears the conjugate(s) while the membrane on the other side overlaps with another membrane bearing the conjugate (s).

12. A method to detect and/or quantify at least one analyte or analyte analogue in a test sample by detecting the presence or absence of said at least one analyte or analyte analogue in a sample put in contact with a sheet-like chromatographic device (1) according to any of claims 1 to 11.
